# EUROPEAN PATENT SPECIFICATION

(11) **EP 3 784 704 B1**
(45) Date of publication and mention of the grant of the patent: **07.06.2023**
(21) Application number: 19795064.5
(22) Date of filing: 02.04.2019
(51) Int. Cl.: C08F 4/14, C10G 50/02, C10M 107/10, C10M 171/02, C08F 110/14, C08F 210/14

(54) **SYNTHETIC FLUIDS WITH IMPROVED BIODEGRADABILITY**
SYNTHETISCHE FLÜSSIGKEITEN MIT VERBESSERTER BIOLOGISCHER ABBAUBARKEIT
FLUIDES SYNTHÉTIQUES À BIODÉGRADABILITÉ AMÉLIORÉE

(30) Priority: 25.04.2018 US 201815962151
(43) Date of publication of application: 03.03.2021
(73) Proprietor: Ineos Oligomers USA LLC, League City TX 77573 (US)
(72) Inventor: BAGHERI, Vahid, League City, TX 77573 (US); MOORE, Lionel, D., Pearland, TX 77584 (US); SANCHEZ-RIVAS, Michel, 7181 Arquennes (BE)
(74) Representative: King, Alex
(86) International application number: PCT/US2019/025456
(87) International publication number: WO 2019/212674

(56) References cited:
- WO-A1-2019/014540
- WO-A2-2018/089457
- GB-A- 2 307 243
- US-A- 5 068 487
- US-A1- 2004 119 046
- US-A1- 2004 129 603
- US-A1- 2013 090 273

## Description

### BACKGROUND OF THE INVENTION

### 1. Technical Field

The present invention relates to low viscosity polyalphaolefin (PAO) compositions characterized by high biodegradability, low pour point, high oxidative stability, and low sludge forming tendencies. More particularly, the invention provides a PAO composition having an improved kinematic viscosity at 100°C and an improved process for the selective production of the improved composition.

### 2. Description of the Prior Art

Oligomers of alpha olefins (also known as linear alpha olefins or vinyl olefins), processes for their production and their use in the formulation of synthetic and semi-synthetic lubricants are known in the art. A few representative methods for making PAO oligomers include the following U.S. Patent Numbers: 3,682,823; 3,763,244; 3,769,363; 3,780,123; 3,798,284; 3,884,988; 3,097,924; 3,997,621; 4,045,507; and 4,045,508.

Traditionally, the alpha olefin oligomers that have proved useful as synthetic base fluids are prepared mainly from linear terminal olefins containing about 8-14 carbon atoms such as 1-octene, 1-decene, 1-dodecene, 1-tetradecene and mixtures thereof. One of the most widely used alpha olefins is 1-decene which can be used alone or in a mixture with other alpha olefins. When linear alpha olefins are employed, the oligomer products comprise mixtures which include varying amounts of dimer, trimer, tetramer, pentamer and higher oligomers. The oligomer products are typically hydrogenated to improve thermal and oxidative stability and must be further fractionated to be useful for specific applications. Such hydrogenated and fractionated oligomer products are known for their superior performance, long use-life, low volatility, low pour points, and high viscosity indexes. Desirably, there would be a base fluid composition that has improved properties, including biodegradability, and would require a simpler production process.

In a conventional polyalphaolefin process, product kinematic viscosities can be adjusted by either removing or adding higher or lower oligomers to provide a composition having the desired viscosity for a particular application. Viscosities in the range of 2 to 150 centistokes (cSt) at 100°C are commonly required, with preferred viscosities being in the range of 2 to 10 cSt.

These low viscosity fluids are especially useful in energy saving applications such as engine lubricating oil to minimize friction and thus improve fuel economy. Used either alone or as blends with mineral oil they can, for example, provide lubricating oils with viscosities which qualify as SAE OW-XX or SAE 5W-XX crankcase oils.

A particularly large market exists for synthetic lubricant base stocks having kinematic viscosity of 2.5 to 4.5 cSt at 100°C especially if this property is combined with low Noack volatility, low pour point, useful low temperature viscosity, and high viscosity index. The 4 cSt PAO can be made by way of decene oligomerization of a 1-decene using a Friedel-Crafts catalyst such as BF3 with a promoter such as an alcohol.

However, 1-decene is in limited supply because it is a co-product made together with a broad range of other alpha olefins. It would be desirable to provide more flexibility in making synthetic base stocks using a broader range of alpha olefins while producing oligomers having substantially similar viscometric properties.

An additional problem associated with making oligomer oils from 1-decene or other alpha olefins is that the oligomer product mix usually must be fractionated into different portions to obtain oils of a given viscosity (e.g. 2, 4, 6, or 8 cSt at 100°C). The commercial production method described above provides an oligomer product mix which, when fractionated, produces the relative amounts of each viscosity product which correspond to market demand. As a result, an excess of one product is often produced in order to obtain the needed amount of the other.

Moreover, while commercially available PAO products provide a useful balance of properties, applications requiring a narrow range of specific viscosities, e.g., 4 cSt material (mainly decene trimer or C₃₀), must be distilled from a complex oligomer mixture. The distillation process, in turn, creates a heavier co-product. Because of this, decene derived PAOs with viscosity between 4 and 5.5 cSt must be post-blended from 4 cSt PAO and a higher viscosity PAO.

It is therefore desirable to produce 2.5 - 4.5 cSt compositions having similar or better properties compared to decene-based oils from feed stocks other than decene - due to the limited decene supply. It is also desirable to produce the aforesaid 2.5 - 4.5 cSt compositions selectively and without any co-products or the need for post-blending.

Polyalphaolefins derived from other linear alphaolefins are known in the art. Polyalphaoleifns derived from pure tetradecene are known to have particularly higher pour point and -40°C viscosity properties.

For polyalphaolefin trimers prepared under identical conditions, it has been demonstrated that the viscosity index increases with increasing chain length of the starting alphaolefin. Conversely, volatility decreases with increasing chain length of the starting alphaolefin. Given that high viscosity index and low volatility are desirable properties for a lubricant, a method of using long carbon number alphaolefins in the manufacture of polyalphaolefins has long been sought.

Unfortunately, it is appreciated that pour point and low temperature viscosity also increase with increasing chain length of the starting alphaolefin. For example, decene (C₁₀) polyalphaolefin trimers are fluid to less than minus 65 degrees Celsius fluid. In contrast, tetradecene (C₁₄) polyalphaolefin trimers in contrast are frozen solid at -20 degrees Celsius according to one source¹.¹ Source: "Synthetic Lubricant Base Stock Processes and Products" Margaret M. Wu, Suzzy C. Ho, and T. Rig Forbus

**TABLE 1: PAO Properties per Margaret Wu, Et Al.**

| | | Kinematic Viscosity, cSt, at | | | | |
|---|---|---|---|---|---|---|
| Name | Carbon Number | 100°C | 40 °C | -40 °C | Viscosity Index | Pour Point, °C |
| Decene trimers | C30 | 3.7 | 15.6 | 2,070 | 122 | <-55 |
| Undecene trimers | C33 | 4.4 | 20.2 | 3,350 | 131 | <-55 |
| Dodecene trimers | C36 | 5.1 | 24.3 | 13,300 | 144 | -45 |
| Tetradecene trimers | C42 | 6.7 | 33.8 | Solid | 157 | -20 |

Shubkin has shown that even for tetradecene oligomers containing a significant amount of tetradecene dimer, the compositions are frozen solid between -18 and -20 degrees Celsius
² .² Shubkin et. Al., "Tailor Making Polyalphaolefins" in Engine oils and Automo tive Lubrication; ed. Wilfred Bartz, 1993, pub Verlag

**TABLE 2: The effect of olefin chain length on the product properties (from Shubkin Table 2.5.9):**

| Starting Olefin | Dimer (Wt %) | KV100°C | KV40°C | KV -18°C | KV -40°C | Point Pour, °C | Flash Point, °C | NOACK |
|---|---|---|---|---|---|---|---|---|
| Octene | 1.2 | 2.77 | 11.2 | 195 | 1320 | <- 65 | 190 | 55.7 |
| Decene | 0.3 | 4.10 | 18.7 | 409 | 3031 | <- 65 | 228 | 11.5 |
| Dodecene | 15.7 | 4.94 | 23.3 | 534 | - | - 48 | 235 | 11.4 |
| Dodecene | 2.8 | 5.70 | 27.8 | 703 | - | -45 | 256 | 3.5 |
| Tetradecene | 40.9 | 5.34 | 24.3 | 519 | Solid | - 24 | 230 | 8.5 |
| Tetradecene | 11.1 | 6.91 | 36.2 | 966 | Solid | - 21 | 250 | 3.7 |
| Tetradecene | 3.0 | 7.59 | 41.3 | 1150 | Solid | -18 | 272 | 2.3 |

It is known that in order for a base stock to be suitable for use in SAE 5W motor oils, the oil must be fluid at -30 degrees Celsius to meet cold cranking requirements. The requirement for SAE 0W oils is even more severe - the base stock must be fluid at -35 degrees Celsius to meet cold cranking requirements.

Many industrial oils also have a -40 degree Celsius Brookfield viscosity requirement that would preclude the use of any base stock with poor low temperature fluidity, such as those derived from tetradecene.

Prior art methods of producing PAOs that are suitable for use as base fluid compositions suffer from numerous deficiencies, so that there remains a need for more improved PAO compositions and improved methods for making said polyalphaolefins. The present invention addresses these needs by providing a single-step low viscosity polyalphaolefin production process and composition characterized by low pour point, good low temperature viscometrics, high viscosity index, and good biodegradability.

### SUMMARY OF THE INVENTION

The present invention claims low viscosity polyalphaolefin (PAO) compositions characterized by high biodegradability, low pour point, high oxidative stability, and low sludge forming tendencies and more particularly provides a PAO composition having a kinetic viscosity at 100°C in the range of about 2.5 to 4.5 cSt.

The invention also claims an improved process for the selective production of the aforesaid composition by oligomerization of 1-tetradecene in the absence or presence of a second comonomer using a catalyst system comprising BF₃ and an alcohol alkoxylate promoter. The process of the invention has a C₂₈ dimer (or co-dimer) selectivity of greater than 70% and does not require further fractionation (after removal of residual unreacted monomer) in order to selectively produce the preferred product. The claimed invention also relates to compositions useful for ultra-low viscosity engine oil application (e.g., 0W-16), and in blended compositions with Group III oils and GTLs improving low temperature properties of such formulations.

The claimed invention further includes a process for the production of a significantly biodegradable low viscosity oil useful as lubricant for environmentally sensitive applications such as marine lubrication and the like. The properties of said low viscosity oil may be further enhanced by the use of conventional lubricant additives in total amounts of up to about 35 weight percent, and preferably from 0.1 to 30 weight percent. Such additives include, for example, dispersants, anti-oxidants, anti-wear agents, anti-foam, corrosion inhibitors, detergents, seal swell agents and viscosity index improvers. These types of additives are well known in the art. While such additives may themselves be substantially biodegradable, though this is not a requirement.

Preferred embodiments of the above low viscosity oil additives include zinc dialkyl-dithiophosphates, calcium aryl sulfonates, overbased calcium aryl sulfonates, barium phenates, barium oxide-neutralized reaction products of phosphorus pentasulfide and terpenes of high molecular weight olefins, hindered alkyl phenols, methylene-bis-dialkyl phenols, dibutyl tin sulfide, dibutyl hydrogen phosphonate, tri-cresyl-phosphate, high molecular weight alkyl succinimides of ethylene-polyamines such as tetraethylene-polyamine, sulfur-bridged alkyl phenols, sulfurized fatty acid esters and amides, silicones and dialkylesters. Proprietary combinations of such additives, "additive packages," which are tailored for specific base oils and applications, are commercially available from several sources including Afton Corporation, Lubrizol or Infineum. Viscosity Index (VI) improvers are separately available.

The fluids of the invention can be used with other base oils or additive oils. These base oils include, but are not limited to, C₆ -C₁₀ 1-olefin based PAO's, mineral oils, or synthetic esters

The over-all biodegradability of the formulations containing the fluids of the invention will depend upon the total functional fluid or lubricant blends including the lubricant additives. Finally, the invention claims the use of the subject polyalphaolefin fluids as a base fluid, either alone or with other lubricating oils (Group I, Group II, Group III, Group III+mineral oils, GTL for instance) in SAE 0W-8, SAE 0W-12, and SAE 0W-16 viscosity grade engine oils or in marine lubricants.

### DESCRIPTION OF THE PREFERRED EMBODIMENT(S)

A polyalphaolefin fluid is claimed which comprises an oligomer prepared from the oligomerization of one or more alpha olefin components. In a preferred embodiment, the alpha olefin component includes a first olefin comprising C₁₄ alphaolefin and from 0 weight percent to about 70 weight percent of a second olefin having an even carbon number between C₁₂ and C₂₀. The structure of the second olefin is shown below where R' is hydrogen (H) and R" is a C₁₀ to C₁₈ hydrocarbyl or where R' and R" vary independently and are both hydrocarbyl and where the sum of R' and R" is C₁₀ to C₁₈.

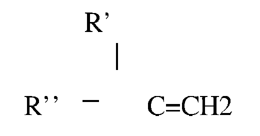

The fluid of the invention is produced by the cationic oligomerization of the olefin or olefins described above using a boron trifluoride catalyst and an alcohol alkoxylate (Rₐ-O-CHR_{b}-CHR_{c}-O-)ₙ-H co-catalyst or "promoter" to yield a fluid that, following hydrogenation, has a kinematic viscosity of from about 2.5 to about 4.6 mm²/s at 100° C and has biodegradability, as determined by the OECD 301B test of at least about 50 percent. The OECD 301B biodegradation test (modified Sturm test) has been recommended by an OECD Expert Group on Degradation to determine the biodegradability of organic chemicals in the aquatic environment. The test is adequate for soluble and insoluble non-volatile organic compounds and measures the amount of CO₂ evolved, thus providing an indication of "complete" biodegradation. The test material is introduced into a flask containing mineral substrate and a bacterial inoculum. After ultrasonic vibration, the flask contents are aerated with CO₂-free air. A control (e.g. sodium benzoate 20 mg C/L) is run in parallel. The EPA recommends the use of aniline (freshly distilled) as a control substance. Any CO₂ released is absorbed in flasks containing a solution of barium hydroxide, whose concentration is periodically determined by titration with hydrochloric acid. Biodegradation is expressed as a percentage of the total amount of CO₂ evolved during the test (corrected for the control), against the theoretical CO₂ that the test material could have produced. Normally the test lasts 28 days, although it can be ended sooner, i.e. as soon as the biodegradation curve has reached a plateau for at least 3 determinations. On the other hand, the test can be extended beyond 28 days if the curve shows that biodegradation has started within the first 28 days, but that the plateau has not been reached by day 28.

For the alcohol alkoxylate (Rₐ-O-CHR_{b}-CHR_{c}-O-)ₙ-H, Rₐ is hydrocarbyl containing from 1 to 24 carbons, including mixtures thereof, R_{b} and R_{c} are independently hydrogen, methyl, or ethyl, and n averages 1 to 15. Preferred alcohol alkoxylate promoters include 2-methoxyethanol and 1-methoxy-2-propanol.

Also claimed is a process for making a polyalphaolefin fluid from a first olefin comprising 1-tetradecene and 0-70 weight percent of a second olefin (R'R"C=CH₂ as described above) comprising contacting the olefin (s) with a catalyst consisting of boron trifluoride and an alcohol alkoxylate (Rₐ-O-CHR_{b}-CHR_{c}-O-)ₙ-H as described above) so as to produce an oligomer reaction product which contains at least about 70 weight percent dimer (or co-dimer) of said olefin monomer(s) and which has a dimer to trimer ratio of greater than about 1. In a preferred embodiment, the 1-tetradecene of this embodiment comprises greater than 90% of the polyalphaolefin mixture.

In a preferred embodiment of the invention, the second olefin comprises a dimer of C₈ alpha olefin, namely 2-n-hexyl-1-decene, which is prepared by contacting C₈ alpha olefin with an aluminum alkyl catalyst such as triethyaluminum and the like as described in US Patent No. 8,455,416, the disclosure of which is incorporated herein by reference. Alternatively, the dimer is prepared by contacting C₈ alpha olefin with a Periodic Group IVB metallocene catalyst activated with organoaluminum compounds and hydrocarbylboron compounds as described in US 6,548,723, the disclosure of which is incorporated herein by reference.

The polyalphaolefin fluid of the invention is prepared without any co-products and without post-blending or overhead distillation of any component other than unreacted monomers.

The polyalphaolefin fluid of the invention is suitable for use as a base fluid, either alone or with other petroleum derived mineral lubricating oils (i.e. Group I, Group II, Group III, Group III+mineral oils), synthetic oils derived from methane using Fischer Tropshe catalysis (i.e. Gas to Liquids fluids or Coal to Liquids fluids for instance) in SAE 0W-8, 0W-12, and SAE 0W-16 viscosity grade engine oils or in marine lubricants meeting Vessel General Permit (VGP) requirements.

A preferred embodiment of the invention provides a hydrogenated composition having a viscosity at 100°C of about 4 cSt, a Noack 250°C volatility weight loss of less than 19% and a Noack 200°C volatility of less than 4%, a Viscosity Index of greater 125, a Pour Point lower than -35 C, and a Cold Crank Simulator viscosity @-35°C of less than 1,500 cP. A more preferred embodiment of the invention provides a hydrogenated composition having a viscosity at 100°C of about 4 cSt, a 250°C Noack volatility weight loss of 13.9%, a Pour Point of approximately -59°C, and Cold Crank Simulator viscosity @-35°C of less than 1,700 cP.

Another embodiment of the invention provides a hydrogenated composition having a viscosity at 100°C of about 3.4 cSt, a Noack 200°C volatility weight loss of 4%, a Pour Point of approximately -50°C, and a Brookfield viscosity @-40° of less than 1,750 cP.

The polyalphaolefins of the present invention combine excellent low temperature characteristics and exceptional high temperature stability, with significant biodegradability. These polyalphaolefins are suitable for use as a base fluid for a wide variety of environmentally friendly lubricants either as the sole base fluid or in combination with vegetable or synthetic esters, other polyalphaolefins (PAOs), mineral oils, gas to liquids fluids, additives, and the like.

Environmentally friendly oils where the fluid of the invention can be used to advantage include, but are not limited to:
2-Cycle Engine Oils
Air tool Lubricants
Chain and Cable Lubricants
Elevator Lubricants
Concrete and Asphalt Release Fluids
Rail Track Greases
Multi-purpose Greases
Hydraulic Fluids for stationery or mobile equipment
Machine Oils
Metal Foundry Mold Release Fluids
Rock Drill Oils
Transformer and transmission line cooling fluids
Slide Way Lubricants
Total Loss Lubricants
Wire rope lubricants
Stem tube lubricants
Forestry oils

Oils that are not necessarily considered to be environmentally friendly but where the fluid of the invention can also be used to advantage include, but are not limited to:
Industrial Gear Lubricants
Transportation Gear Lubricants
Compressor Oils
Gasoline Engine Crankcase Lubricants
Diesel Engine Crankcase Lubricants
Transmission Oils
Turbine Oils

In particular, the polyalphaolefins of the invention meet the viscometric requirements defined by the Society of Automotive Engineers (SAE) for use as base fluids in 0W-8, 0W-12, and 0W-16 engine oils.

Moreover, the polyalphaolefins of the invention may be used in any lubricant type where vegetable esters, estolides, or natural/synthetic ester derivatives find application. Marine stern tube lubricants, open gear oils, wire rope oils, forestry oils, and hydraulic fluids are all applications which can benefit from the addition of the polyalphaolefins of this invention. Oils in this category can be made to be significantly biodegradable by the proper choice of additives and thickeners.

One preferred embodiments of formulated fluids according to the foregoing include mixtures of a) from 1-97% of the polyalphaolefin fluid of the invention mixed with b) 0 - 60% of fluids selected from the group consisting of synthetic esters, synthetic hydrocarbon fluids, mineral oils, natural esters, or hydrocarbon oils derived from natural and petroleum sourced raw materials, and c) 0.1-30% of additives such as dispersants, anti-oxidants, anti-wear agents, anti-foam agents, corrosion inhibitors, detergents, seal-swell agents, viscosity improvers and combinations thereof, such that the overall composition meets the viscometric requirements of the SAE OW-XX grade, where XX is 16 or lower.

Another preferred embodiment of said functional fluid or lubricant composition includes a mixture comprising a) from 1-97% of the polyalphaolefin fluid of the invention mixed with b) 0 - 60% of fluids selected from the group consisting of synthetic esters, synthetic hydrocarbon fluids, mineral oils, natural esters, or hydrocarbon oils derived from natural and petroleum sourced raw materials, and c) 0.1 to 70% of additives such as dispersants, anti-oxidants, anti-wear agents, anti-foam agents, corrosion inhibitors, detergents, seal-swell agents and viscosity improvers, such that the overall composition exhibits biodegradability, as determined by the CEC L-33 A94 test (or equivalent) of at least 50% where said composition may be intentionally or unintentionally released into the environment.

In another preferred embodiment functional fluid or lubricant composition is provided, wherein the composition comprises a) 1-98% of the polyalphaolefin fluid of the invention mixed with b) 0 - 60% of one or more components chosen from natural or synthetic esters, natural or synthetic hydrocarbon fluids, or hydrocarbon oils derived from natural and petroleum sourced raw materials, and c) contains 0.1 to 70% of additives such as dispersants, anti-oxidants, anti-wear agents, anti-foam agents, corrosion inhibitors, detergents, seal-swell agents and viscosity improvers This embodiment may be especially preferred in applications including the operation of a chain saw, an outboard motor, farm equipment, earth moving equipment, or marine or sub-marine fluids. In marine-specific applications the overall composition can be tailored to meet the requirements of the EPA Vessel General Permit mandates.

### Examples

Commercially produced 1-tetradecene (C₁₄) from INEOS Oligomers was used as received; it can be substituted by 1-tetradecene from other suppliers. All process steps can be conducted in batch, semi-batch, or in a continuous manner. The reaction can be run in continuous mode employing 2-5 continuous stirred tank reactors (CST) in series or parallel or a combination thereof.

### Comparative Example 1:

To a 1-gallon Parr reactor equipped with jacketed heating and internal cooling was charged with 1,200 g 1-tetradecene and 3.0 g 1-butanol (1-BuOH) and was heated to 50°C with stirring. Boron trifluoride (BF₃) was introduced as a gas and it was adjusted to a steady state pressure of 40 psig. The reaction was stirred for 90 minutes. The reaction mixture was quenched with 400 ml of 8% NaOH and then washed with distilled water. Removal of a residual unreacted monomer as a volatile overhead fraction under reduced pressure (220°C, 0.1 mmHg) resulted in the isolation of 837.1 g of a clear fluid as the bottoms fraction which was hydrogenated under a set of standard hydrogenation conditions (at 170°C, 400 psi hydrogen, using Ni on Kieselguhr catalyst) to produce a synthetic basestock having the following properties:

**TABLE 1: Properties of Comparative Example 1**

| Analysis | Units | Test Method | Properties |
|---|---|---|---|
| KV 100°C | mm²/S | ASTM D-445 | 4.70 |
| KV 40°C | mm²/S | ASTM D-445 | 21.3 |
| VI | --- | ASTM D-2770 | 145 |
| Pour Point | °C | ASTM D-97 | -27 |
| Bromine number | g/100g | IP-129 | 0.1 |

### Invention Example 2:

To a 1-gallon Parr reactor equipped with jacketed heating and internal cooling was charged with 1,400 g 1-tetradecene and 4.2 g of 1-methoxy-2-propanol (1-MOP) and was heated to 50°C with stirring. Boron trifluoride (BF3) was introduced as a gas and it was adjusted to a steady state pressure of 20 psig. The reaction was stirred for 120 minutes. The reaction mixture quenched with 400 ml of 8% NaOH and then washed with distilled water. Removal of the residual unreacted monomer as a volatile overhead fraction under reduced pressure (220°C, 0.1 mmHg) resulted in isolation of 1,063.1 g of a clear fluid as the bottoms fraction which was hydrogenated under a set of standard hydrogenation conditions (at 170°C, 400 psi hydrogen, using Ni on Kieselguhr catalyst) to produce a synthetic basestock of the invention having the following properties:

**TABLE 2: Properties of INVENTION Embodiment A**

| Analysis | Units | Test Method | Properties |
|---|---|---|---|
| KV 100°C | mm 2/S | ASTM D-445 | 3.89 |
| KV 40 °C | mm²/s | ASTM D-445 | 16.68 |
| VI | --- | ASTM D-2770 | 130 |
| Pour Point | °C | ASTM D-97 | -42 |
| Flash Point | °C | ASTM D-92 | 224 |
| Noack, 250°C | % wt loss | CEC L40-A-93 | 16.4 |
| Noack, 200°C | % wt loss | CEC L40-A-93 | 2.1 |
| Appearance | --- | Observation | Clear & Bright |
| % Transmittance@ 440 nm | % | INEOS | 99 |

The table above shows that once residual unreacted monomer removed, the resultant PAO has inventive balance of viscometric properties, it is a straight run single recipe 4 cSt fluid produced without further distillation. It is also a 4 cSt fluid with high Viscosity Index, good Noack volatility, and has significantly improved Pour Point properties comparing to conventional C14 oligomer made by BF3 catalyst system promoted by the traditional alcohols like 1-butanol (1-BuOH):

**TABLE 3: Pour Point of INVENTION Embodiment A vs Comparative Example 1**

| Examples | Promoter | Pour Point, °C |
|---|---|---|
| Comparative Example #1 | 1-BuOH | -27 |
| Invention Example #2 | 1-MOP | -42 |

Oligomer composition of the PAO of the Invention from Example 2 by GC showed the following composition:
C₂₈ (dimer): 88.0 area%
C₄₂₊ (trimer and higher): 12.0 area%

### Invention Example 3:

The procedure set out in Example 2 was run on a pilot scale. The product had the following properties:

**TABLE 4: Properties of INVENTION Embodiment A**

| Analysis | Units | Test Method | Properties |
|---|---|---|---|
| KV 100°C | mm²/S | ASTM D-445 | 3.93 |
| KV 40 °C | mm²/S | ASTM D-445 | 16.37 |
| VI | - | ASTM D-2770 | 140 |
| Pour Point | °C | ASTM D-97 | -39 |
| Flash Point, COC | °C | ASTM D-92 | 213 |
| Noack, 250°C | % wt loss | CEC L40-A-93 | 15.8 |
| Appearance | | Observation | Clear & Bright |
| % Transmittance@ 440 nm | | INEOS | 99 |
| Water | ppm | ASTM D-3401 | <25 ppm |
| Cold Cranking Simulator Viscosity @ - 30 °C | mPa.S | ASTM D-5293 | 821 |
| Cold Cranking Simulator Viscosity @ - 30 °C | mPa.S | ASTM D-5293 | 1,335 |
| Density @15.6 °C | g/ml | ASTM D-4052 | 0.820 |
| Bromine number | g/100g | IP-129 | 0.1 |
| Biodegradation | % | OECD 301B | 54%, 28 days |

### Invention Example 4:

To a 1-gallon Parr reactor equipped with jacketed heating and internal cooling was charged with 1,200 g 1-tetradecene and 3.1 g 2-methoxyethanol (2-MOE) and was heated to 50°C with stirring. Boron trifluoride (BF3) was introduced as a gas and it was adjusted to a steady state pressure of 40 psig. The reaction was stirred for 90 minutes. The reaction mixture quenched with 400 ml of 8% NaOH and then washed with distilled water. Removal of residual unreacted monomer as a volatile overhead fraction under reduced pressure (220°C, 0.1 mmHg) resulted in isolation of 737.3 g of a clear fluid as the bottoms fraction which was hydrogenated under a set of standard hydrogenation conditions (at 170°C, 400 psi hydrogen, using Ni on Kieselguhr catalyst) to produce a synthetic basestock having the following properties:

**TABLE 5: Properties of INVENTION Embodiment A1**

| Analysis | Units | Test Method | Properties |
|---|---|---|---|
| KV 100°C | mm²/S | ASTM D-445 | 3.75 |
| KV 40 °C | mm²/S | ASTM D-445 | 15.71 |
| VI | - | ASTM D-2770 | 130 |
| Pour Point | °C | ASTM D-97 | -39 |
| Bromine number | g/100g | IP-129 | 0.2 |

### Invention Example 5:

To a 1-gallon Parr reactor equipped with jacketed heating and internal cooling was charged with 476 g of 1-tetradecene, 924 g 1-dodecene, and 4.2 g 1-methoxy -2-propanol (1-MOP) and was heated to 50°C with stirring. Boron trifluoride (BF3) was introduced as a gas and it was adjusted to a steady state pressure of 20 psig. The reaction was stirred for 120 minutes. The reaction mixture was quenched with 400 ml of 8% NaOH and then washed with distilled water. Removal of residual unreacted monomer fraction as a volatile overhead fraction under reduced pressure (220°C, 0.1 mmHg) resulted in isolation of 993.9 g of a clear fluid as the bottoms fraction which was hydrogenated under a set of standard hydrogenation conditions (at 170°C, 400 psi hydrogen, using Ni on Kieselguhr catalyst) to produce a synthetic basestock having the following properties:

**TABLE 6: Properties of INVENTION Embodiment B**

| Analysis | Units | Test Method | Properties |
|---|---|---|---|
| KV 100°C | mm²/S | ASTM D-445 | 3.43 |
| KV 40 °C | mm²/S | ASTM D-445 | 14.14 |
| VI | --- | ASTM D-2770 | 119 |
| KV -40 °C | mm²/S | ASTM D-445 | 1,898 |
| Pour Point | °C | ASTM D-97 | -50 |
| Flash Point, COC | °C | ASTM D-92 | 210 |
| Noack, 200°C | % wt loss | CEC L40-A-93 | 4.0 |
| Appearance | --- | Observation | Clear & Bright |
| % Transmittance @ 440 nm | % | INEOS | 99 |
| Water | ppm | ASTM D-3401 | 10 |
| Brookfield Viscosity @ -40 °C | mPa.S | ASTM D-2983 | 1,709 |
| Density @15.6 °C | g/ml | ASTM D-4052 | 0.8188 |
| Bromine number | g/100g | IP-129 | 0.1 |
| Biodegradation | % | OECD 301B | 87.3%, 28 days |

### Invention Example 6:

Example 4 was repeated using 2-methoxyethanol (2-MOE) as the co-catalyst. The product had the properties shown in Table 6.

**TABLE 7: Properties of INVENTION Embodiment B**

| **Analysis** | **Units** | **Method** | **Results** |
|---|---|---|---|
| KV 100°C | mm²/s | ASTM D-445 | 3.28 |
| KV 40 °C | mm²/s | ASTM D-445 | 13.48 |
| KV - 40°C | mm²/s | ASTM D-445 | 1,318 |
| Pour Point | °C | ASTM D-97 | -54 |
| Brookfield Viscosity @ - 40°C | mPa·S | ASTM D-2983 | 1,260 |
| Cold Cranking Simulator Viscosity @ -35°C | mPa·S | ASTM D-5293 | 920 |
| Flash Point, PMCC | °C | ASTM D-93 | 210 |
| Flash Point, COC | °C | ASTM D-92 | 216 |
| Noack @ 200°C | %wt | CEC L40-A-93 | 5.1 |
| Water | ppm | ASTM D-3401 | 17 |
| Total Acid Number | mgKOH/g | ASTM D-974 | 0.001 |
| Density 15°C | g/ml | ASTM D-4052 | 0.8165 |
| Refractive Index | - | ASTM 0-1218 | 1.4541 |
| Bromine number | g/100g | IP-129 | 0.2 |

### Invention Example 7:

To a 1-gallon Parr reactor equipped with jacketed heating and internal cooling was charged 515.0 g 1-tetradecene, 885.0 g 2-n-hexyl-1-decene (the dimerization product of 1-octene as per US 8,455,416), and 1.4 g 1-methoxy-2-propanol (1-MOP). The mixture was heated to 30°C with stirring. Boron trifluoride (BF3) was introduced as a gas and it was adjusted to a steady state pressure of 20 psig. The reaction was stirred for 90 minutes. The reaction mixture was quenched with 400 ml of 8% NaOH and then washed with distilled water. Removal of residual unreacted monomer as volatile overhead fraction under reduced pressure (220°C, 0.1 mmHg) resulted in isolation of 1,162.0 g of a clear fluid as the bottoms fraction which was hydrogenated under a set of standard hydrogenation conditions (at 170°C, 400 psi hydrogen, using Ni on Kieselguhr catalyst) to produce a synthetic basestock having the following properties:

**TABLE 7: Properties of INVENTION Embodiment C**

| Analysis | Units | Test Method | Properties |
|---|---|---|---|
| KV 100°C | mm²/S | ASTM D-445 | 4.07 |
| KV 40°C | mm²/S | ASTM D-445 | 18.18 |
| KV -40 °C | mm²/S | ASTM D-445 | 2705 |
| VI | - | ASTM D-2770 | 126 |
| Pour Point | °C | ASTM D-97 | -57 |
| Bromine number | g/100g | IP-129 | 0.1 |
| Biodegradation | % | OECD 301B | 100%, 28 days |

### BIODEGRADABILITY OF POLYALPHAOLEFINS OF THE INVENTION

Substances are considered to be biodegradable if they can be efficiently decomposed by microorganisms. There are a number of procedures that can, and have been, used to assess biodegradability. One test protocol that has been approved by the OECD and that is well suited to the study of paraffinic hydrocarbons is the 301B "Ready Biodegradability" test (also known as the modified Stürm test).

### Example 8:

In the OECD 301B test, the test sample is exposed to activated sewage sludge and a culture medium in sealed tubes that are held at 21 °C. The degradation of the test material is assessed by measuring the carbon dioxide produced over a 28 day period. Control solutions containing the sewage micro-organisms and standard materials are also run.

Using this test, the polyalphaolefin of Example 7 attained 100% degradation after 28 days and 60% degradation after 10 days.

In contrast, an equiviscous 1-decene derived PAO attained only 28% degradation after 28 days. Other comparisons can be found in Table 8 below:

**Table 8: Biodegradability of Example 7 (INVENTION Embodiment C)**

| | Biodegradability (%); OECD 301 B¹ 28 days | Biodegradability (%); OECD 301B 10 days² | Nominal Kinematic Viscosity at 100°C (ASTM D-445) |
|---|---|---|---|
| INVENTION Example 7 | 100 | ≥60 | 4 cSt |
| Decene Based | 28 | <60 | 4 cSt |
| PAO4 | | | |
| *Durasyn 164* | | | |
| Decene Based | 17 | <60 | 6 cSt |
| PAO6 | | | |
| *Durasyn 166* | | | |
| Vegetable Esters | 70 - 100 | ND | --- |

| | | | |
|---|---|---|---|
| 1. The OECD 301B test, also known as the Modified Sturm test, measures CO₂ released from the test bottle as biodegradation progresses. The theoretical CO₂ value is calculated by assuming that all carbon in the test compound is metabolized to CO₂. The test is run for 28 days. 2. The "Readily Biodegradable" criterion requires production of at least 60% of the CO₂ that theoretically could be produced within ten days of reaching the 10% biodegradation threshold. Because microbes will use some carbon to grow new microbes, not all of the theoretical value of CO₂ will be produced. | | | |

### Example 9

The product of Invention Example 3, a polyalphaolefin derived entirely from 1-tetradecene attained 54% degradation in the OECD 301B biodegradation test after 28 days.

### Example 10

The product of Invention Example 5, a polyalphaolefin derived from 1-tetradecene and 1-dodecene attained 87.3% degradation in the OECD 301B biodegradation test after 28 days.

### FORMULATED OILS CONTAINING THE INVENTION

The fluids of the present invention can be used in formulated lubricant products that are generally not designed to be substantially biodegradable such as crankcase lubricants, industrial and automotive gear oils, transmission oils, and the like but for which accidental discharge to the environment is possible.

Because of the unique balance of biodegradability, low temperature properties, and thermal-oxidation stability, the fluids of the invention are especially well suited for use in lubricants where discharge to the environment can be anticipated either accidentally (e.g. marine lubricants) or on purpose (e.g. forestry lubricants). For these lubricant classes, the significant biodegradability of the fluids of the invention is a plus.

In lubricant products formulated to be substantially biodegradable, such as hydraulic oils, paper mill oils, chain saw lubricants, outboard motor lubricants, turbine oils, compressor oils, greases, and the like, the fluids of the invention can be used either as the sole base fluid or in combination with other base fluids. Ideally, these other fluids would themselves have fair to good inherent biodegradability. Illustrative fluids that might be used with the fluids of the invention include vegetable oils (e.g. rapeseed oil, canola oil, and the like), vegetable oil derivatives (e.g. estolide esters), or even synthetic dicarboxylic acid esters that have high biodegradability and good viscometric properties.

In applications where high overall biodegradability is not a requirement, the product of the invention can be used with conventional polyalphaolefins (i.e. hydrogenated 1-alkene hydrocarbon liquid oligomers), mineral oils, or gas to liquid fluids if used as a minor components of the total formulation.

Additives are often required to enhance the specific performance attributes of formulated oils that might incorporate the fluids of the invention. These additives may or may not be biodegradable in their own right and include wear inhibitors, detergents, viscosity index improvers, friction modifiers, fuel economy additives, antioxidants or thermal stabilizers, dispersants, extreme pressure agents, tackiness additives, rust inhibitors, wax modifiers, foam inhibitors, copper passivators, sulfur scavengers, seal swell agents, color stabilizers, and like materials.

The fluids of the invention may be used with additives that are themselves designed to be biodegradable.

Where possible, additive should at least be chosen such that they do not substantially interfere with the biodegradability of the overall composition.

### Example 11:

*Demonstration Tractor Fluid incorporating a 3.9 cSt fluid of the INVENTION*

**Table 9**

| Tractor Fluid | Weight % |
|---|---|
| Additive Package¹, Wt% | 7.6 |
| INVENTION 3.9 cSt², Wt% | 81.0 |
| Viscosity Modifier³, Wt% | 11.4 |
| | |
| KV @ 100°C (cSt) | 7.75 |
| KV @ 40 °C (cSt) | 35.0 |
| Viscosity Index | 201 |
| Flash Point | 234 °C |
| Cold Cranking Simulator Viscosity, -20°C (cP) | 650 |
| CEC-L-33-A-94 Biodegradabilty | >78%⁴ |

| | |
|---|---|
| 1. Commercially available tractor hydraulic additive package from Afton; Hitec 8703 2. Fluid of the invention according to Example 7 3. Commercially available shear stable viscosity improver from Evonik; Viscoplex 8-219. 4. This test method provides a procedure to evaluate the comparative biodegradability of two-cycle outboard engine lubricants, or other oils, against the biodegradability of standard calibration materials | |

### Example 12:

*Demonstration Environmentally Friendly Chain Oil incorporating a 3.9 cSt fluid of the INVENTION*

**Table 10**

| 2-Cycle Chain Oil | Weight % |
|---|---|
| Additive Package¹, Wt% | 5.5 |
| INVENTION 3.9 cSt², Wt% | 60.0 |
| Canola Oil³, Wt% | 34.5 |
| | |
| KV @ 100°C (cSt) | 5.8 |
| KV @ 40 °C (cSt) | 26.6 |
| Viscosity Index | 172 |
| Flash Point | 239 °C |

| | |
|---|---|
| 1. Commercially available additive package; Esp Afton Hitec 2211 2. Fluid of the invention according to Example 7 3. Commercially available Canola Oil from Archer Daniels Midland 4. Fluid of the invention according to example | |

### Example 13:

*Demonstration 2 Cycle Marine Oil incorporating the fluid of the INVENTION*

**Table 11**

| 2-Cycle Marine Oil | **Weight %** |
|---|---|
| Additive Package¹, Wt% | 18.3 |
| INVENTION 3.9 cSt², Wt% | 40.0 |
| Synthetic Ester³, Wt% | 34.1 |
| Synthetic Ester⁴, Wt% | 7.6 |
| | |
| KV @ 100 °C (cSt) | 8.1 |
| KV @ 40 °C (cSt) | 46.9 |
| Viscosity Index | 148 |
| Flash Point | 236 °C |
| CEC-L-33-A-94 Biodegradabilty | >70%⁵ |

| | |
|---|---|
| 1. Commercially available additive package meeting NMMA TC W3; Esp Lubrizol 424 2. Fluid of the invention according to Example 7 3. Commercially available saturated linear acid complex synthetic ester; Esp. Nycobase 8306 4. Commercially available partially unsaturated linear acid complex synthetic ester; Esp. Nycobase 4045 5. This test method provides a procedure to evaluate the comparative biodegradability of two-cycle outboard engine lubricants, or other oils, against the biodegradability of standard calibration materials | |

### Example 14:

Demonstration Biodegradable Marine Multigrade Gear Oil/Stern Tube Oil

**Table 12**

| Marine Multigrade Oil | **Weight %** |
|---|---|
| Additive Package¹, Wt% | 9.42 |
| Lexolube CG-3000² | 39.02 |
| Canola Oil³ | 8.49 |
| INVENTION 4.55 cSt⁴, Wt% | 43.07 |
| Estimated % Bio-based Carbon | 34.6 |
| KV @ 100ºC, D445 | 26.10 |
| KV @ 40ºC, D445 | 175.74 |
| Viscosity Index, D2270 | 184 |
| Brookfield Viscosity @ -40°C, D5293 | 381,200 |
| Pour Point, ºC, D97 | -45 |
| Flash Point, ºC, D92 | 226 |
| Total Acid Number | 0.35 |
| Specific Gravity at 15.6°C, D4052 | 0.9020 |
| Bromine Number, IP-129 | 0.12 |
| Noack Volatility, weight percent, 250°C | 11.1 |

| | |
|---|---|
| 1. Commercial biodegradable gear oil package from Functional Products; GA-502 2. Commercial bio-based complex ester thickener; 293 cSt at 100°C, 3,113 cSt at 40°C from Inolex. 67% bio-content; Lexolube CG-3000. 3. Commercially available Canola Oil from Cargill, 8.53 cSt at 100°C, 38.71 cSt at 40°C 4. Fluid of the invention according to Example 2 5. A 75W oil must have a minimum viscosity at 100°C of 24 to 41 mm²/s | |

### Conventional Lubricants

It is anticipated that the synthetic fluids of the current invention will be used wherever hydrogenated 1-decene oligomers of similar viscosity are used. Applications include, but are not limited to, automotive crankcase oils, heavy duty diesel oils, automatic transmission fluids, continuously variable transmission fluids, and industrial and automotive gear oils, compressor/turbine oils and particularly applications benefiting from energy saving features inherent in low viscosity fluids such as low and ultra-low viscosity OW-XX engines oil where X is 16 or less. Several demonstration formulations were devised to illustrate the suitability of the fluids of the invention for a number of 0W-XX formulations.

### Passenger Car Motor Oils

The synthetic fluids made by the present invention are ideally suited for use as components of full synthetic and/or semi-synthetic lubricating oils used in internal combustion engines. The fluids of the invention can be used as the entire base lubricant or can be blended with other lubricating oils including Group I, II, or III mineral oils, GTL (gas to liquid) oils, synthetic ester oils (e.g. di-2-ethylhexyl adipate, trimethylolpropane tripelargonate, etc.), alkyl naphthalene oils (e.g. di-dodecylnapthalene, di-tetradecylnapthalene, etc.) and the like. The lubricating oils used in internal combustion engines are typically formulated to contain conventional lubricating oil additives such as calcium aryl sulfonates, overbased calcium sulfonates, calcium or barium phenates, overbased magnesium alkylbenzene sulfonates, zinc dialkyldithiophosphates, VI improvers (e.g. ethylene-propylene copolymers, polyalkylmethacrylates, etc.), ashless dispersants (e.g. polyisobutylenesuccinimides of tetraethylene pentamine, polyisobutylenephenol-formaldehyde-tetraethylene pentamine Mannich condensation products, etc.), pour point depressants, friction modifiers, rust inhibitors, demulsifiers, oil soluble antioxidants (e.g. hinder phenols or alkylated diphenyl amines), various sulfurized components, and foam inhibitors (antifoams).

As used herein, a synthetic oil or base stock is a lubricant derived from chemical compounds that are artificially made. Synthetic lubricants can be manufactured using chemically modified petroleum components such as ethylene, the starting material from which alpha-olefins are derived, or can be synthesized from other non-petroleum raw materials. Synthetic base stocks, being man-made, usually have controlled molecular structure with predictable properties, unlike mineral base oils, which are complex mixtures of naturally occurring hydrocarbons1. Mineral oils or petroleum-refined oils are defined as API Group I, II, II+, III, and III+ base oil stocks.

API Group III base oils are sometimes considered to be fully synthetic, but as used herein Group III / Group III+ base oils are classified as mineral-base stocks. Lubricants containing synthetic lubricants and no content of API Group I, II, II+, III and III+ mineral-base stocks are considered to be fully synthetic. Lubricants containing synthetic lubricants used in conjunction with API Group I, II, II+, III and III+ base stocks are considered to be "semi-synthetic" or "part synthetic".

In both part synthetic and full synthetic oils, the base stocks are combined with additive packages, individual performance additives, and esters (typically API Group V) or other solubility enhancers.

Proprietary combinations of such additives, called additive packages, are tailored for specific base oils and applications, and are commercially available from several sources including Lubrizol, Infineum, and Afton Corporations. Viscosity Index (VI) improvers are available from these and other suppliers.

The fluid of the invention can be used to formulate OW-XX viscosity grade passenger car motor oils that are desirable for their energy conserving qualities.

### Example 15:

### Passenger Car Demonstration Oil

The following 0W-16 full and part-synthetic passenger car motor oils were formulated containing the fluids of the INVENTION.

**Table 13**

| Full Synthetic 0W-16 PCMO | | | | | |
|---|---|---|---|---|---|
| **Properties** | **0W16** | **0W16** | **0W16** | **0W16 Comparative Examples** | **0W16 Comparative Examples** |
| Additive package¹ | 10.0 | 10.0 | 10.0 | 10.0 | 10.0 |
| Invention 3.9 cSt² | 76.5 | --- | --- | --- | --- |
| Invention 4 cSt³ | --- | 76.5 | --- | --- | --- |
| Invention 3.4 cSt⁴ | --- | --- | 34.6 | --- | --- |
| C12 PAO5⁵ | --- | --- | 41.9 | --- | 41.9 |
| C10 PAO4⁶ | --- | --- | --- | 76.5 | --- |
| Group III Oil, 3.1 cSt⁷ | --- | --- | --- | --- | 34.6 |
| Viscosity Modifier⁸ | 3.5 | 3.5 | 3.5 | 3.5 | 3.5 |
| Ester⁹ | 10.0 | 10.0 | 10.0 | 10.0 | 10.0 |
| | | | | | |
| Visc, cSt, 100°C | 7.0 | 6.8 | 6.95 | 6.9 | 7.0 |
| Visc, cSt, 40°C | 34.2 | 32.5 | 34.18 | 33.0 | 35.4 |
| Viscosity Index | 173 | 175 | 169 | 165 | 164 |
| Flash Point °C | 240 | 228 | 230 | 240 | 236 |
| Pour Pt, °C | -56 | -40 | -45 | -59 | -45 |
| Noack | 9.6 | 13.0 | 13.9 | 10.9 | 16.4 |
| HTHS @ 150°C | 2.30 | 2.30 | 2.30 | 2.32 | N/D |
| CCS @ -35°C | 3,360 | 2,960 | 2,700 | 3,486 | 4,707 |

| | | | | | |
|---|---|---|---|---|---|
| 1. Commercial dispersant/inhibitor package. Infineum P5707, API SN, ILSAC GF5/GF6B 2. Fluid of the invention according to Example 7 3. Fluid of the invention according to Example 3 4. Fluid of the invention according to Example 5 5. Commercial hydrogenated 1-dodecene polyalphaolefin from INEOS; 5.1 cSt at 100°C 6. Commercial hydrogenated 1-decene polyalphaolefin from INEOS; 3.93 cSt at 100°C 7. Commercial Group II mineral oil; Puralub 75, 3.1 cSt at 100°C, 102 VI, -15°C pour point 8. Commercial 15% m/m solution of hydrogenated styrene polyisoprene polymer in PAO6 from Shell; SV261 9. Ester from Croda; P3970, 4.9 cSt at 100°C, 213 VI | | | | | |

### Example 16:

When a 4 cSt Group III mineral oil is replaced by a 4 cSt fluid of the invention in a blend with a 6 cSt Group III oil and a pour point depressant, the resulting blend had lower pour point, lower cold cranking viscosity at -30°C and -35°C and higher flash point.

| | **Invention** | Comparator |
|---|---|---|
| Component | Weight Percent | Weight Percent |
| INVENTION 4 cSt¹ | 49.9 | --- |
| 4 cSt Group III² | --- | 49.9 |
| 6 cSt Group III³ | 49.9 | 49.9 |
| Pour Point Depressant⁴ | 0.2 | 0.2 |
| Viscosity @ 100°C, cSt | 4.82 | 5.12 |
| Viscosity @ 40°C, cSt | 22.85 | 24.93 |
| Viscosity @ -40°C, cSt | --- | --- |
| Viscosity Index | 137 | 139 |
| Pour Point, °C | -39 °C | -30 °C |
| Flash Point, °C (COC) | 235 °C | 229 °C |
| Noack Volatility | 12.9 % | 11.0% |
| Cold Crank -30°C | 1,588 | 2,377 |
| Cold Crank -35°C | 2,746 | 4,380 |

| | | |
|---|---|---|
| 1. Fluid of the invention according to Example 3 2. 4 cSt Group III mineral oil; S-Oil Ultra S4, 4.2 cSt @ 100°C, 125 VI, -21°C pour point. 14.2% Noack volatility 3. 6 cSt Group III mineral oil; S-Oil Ultra S6, 5.9 cSt @ 100°C, 127 VI, -15°C pour point. 8.2% Noack volatility 4. Pour point depressant; Afton Hitec 623 | | |

### Example 17:

Part Synthetic 0W-16 PCMO

| **Properties** | **Invention 0W16** | **0W16 Comparative Example** |
|---|---|---|
| Additive package¹ | 10.0 | 10.0 |
| Group III Plus Oil, 4 cSt² | 46.5 | 76.5 |
| Invention 3.9 cSt³ | 30.0 | --- |
| Viscosity Modifier⁴ | 3.5 | 3.5 |
| Ester⁵ | 10.0 | 10.0 |
| | | |
| Visc, cSt, 100°C | 6.9 | 6.9 |
| Visc, cSt, 40°C | 33.1 | 33.8 |
| Viscosity Index | 167 | 170 |
| Flash Point °C | 234 | 230 |
| Pour Pt, °C | -51 | -48 |
| Noack | 11.4 | 12.0 |
| HTHS @ 150°C | 2.30 | 2.28 |
| CCS @ -35 °C | 3,580 | 3,880 |
| MRV @ -40°C | 14,505 | 18,300 |

| | | |
|---|---|---|
| 1. Commercial dispersant/inhibitor package. Infineum P5707, API SN, ILSAC GF5/GF6B 2. Group III Plus Mineral Oil; Yubase 4+, 4.1 cSt, 134 VI 3. Fluid of the invention according to Example 7 4. Commercial 15% m/m solution of hydrogenated styrene polyisoprene polymer in PAO6 from Shell; SV261 5. Ester from Croda; P3970, 4.9 cSt at 100°C, 213 VI | | |

## Claims

1. A process for the production of a polyalphaolefin fluid having predetermined properties comprising:
a. reacting an alpha olefin component comprising at least one alpha olefin containing from 30 to 100% 1-tetradecene (C₁₄) in the presence of a catalyst system consisting of BF₃ combined with an alcohol alkoxylate promoter to produce a reacted bottom product and unreacted residual monomers;
b. removing the unreacted residual monomers by distillation as an overhead fraction; and
c. hydrogenating at least a portion of said bottom product to obtain a hydrogenated fluid having a 100° C kinematic viscosity, determined according to ASTM D-445 of 2.5 to 4.6 centistokes, and
wherein the polyalphaolefin fluid has a dimer C₂₈ content of greater than 70%.

2. The process of claim 1 wherein the alpha olefin component comprises greater than 90% C₁₄ alpha olefin.

3. The process of claim 1 wherein the hydrogenated polyalphaolefin fluid has a biodegradability, as determined by the OECD 30 IB test of at least 50 percent.

4. The process of claim 1 wherein the alpha olefin component comprises a first alpha olefin and a second alpha olefin, and wherein the first alpha olefin is C₁₄ alpha olefin and the second olefin is C₁₂ alpha olefin.

5. The process of claim 4 where the C₁₄/C₁₂ co-dimer content of the polyalphaolefin fluid is greater than 80%.

6. The process of claim 1 wherein the alpha olefin component comprises a first alpha olefin and a second alpha olefin, and wherein the first alpha olefin is C₁₄ alpha olefin and the second olefin is a dimer of C₈ alpha olefin.

7. The process of claim 6 where the C₁₄/C₁₆ co-dimer content of the polyalphaolefin fluid is greater than 80%.

8. The process of claim 1 wherein the alcohol alkoxylate promoter in step (a) is 2-methoxyethanol.

9. The process of claim 1 wherein the alcohol alkoxylate promoter in step (a) is 1-methoxy-2-propanol.

10. A lubricant composition comprising a hydrogenated polyalphaolefin fluid obtainable by the process of any one of claims 1 to 9 and either
a. having a 100°C kinematic viscosity of 4 cSt, a 250°C Noack volatility weight loss of less than 19% and a 200°C Noack volatility weight loss of less than 4%, a Viscosity Index of greater than 125, a Pour Point lower than -35°C, and Cold Crank Simulator viscosity @-35°C of less than 1,500 cP, or
b. having a kinematic viscosity at 100°C of 3.4 cSt, a 200°C Noack volatility weight loss of 4%, a Pour Point of -50°C, and a Brookfield viscosity @-40° of less than 1,750 cP or
c. having a kinematic viscosity at 100°C of 4 cSt, a 250°C Noack volatility weight loss of 13.9%, a Pour Point of -59°C, and Cold Crank Simulator viscosity @-35°C of less than 1,700 cP.

11. A formulated full or part synthetic lubricating oil comprising a hydrogenated fluid having a 100° C kinematic viscosity of 2.5 to 4.6 centistokes which hydrogenated fluid is a polyalphaolefin fluid obtainable by the process of any one of claims 1 to 9.

12. The formulated lubricating oil of claim 11 comprising a mixture of
a. from 1 to 97 percent of said hydrogenated fluid having a 100° C kinematic viscosity of 2.5 to 4.6 centistokes; and
b. from 0 to 60 percent of fluids selected from the group consisting of synthetic esters, synthetic hydrocarbon fluids, mineral oils, natural esters, or hydrocarbon oils derived from natural and petroleum sourced raw materials and combinations thereof; and
c. from 0.1 to 30 percent of additives selected from the group consisting of dispersants, anti-oxidants, anti-wear agents, anti-foam agents, corrosion inhibitors, detergents, seal-swell agents, viscosity improvers and combinations thereof.

13. The formulated lubricating oil of claim 11 comprising a mixture of
a. from 1 to 97 percent of said hydrogenated fluid having a 100° C kinematic viscosity of 2.5 to 4.6 centistokes; and
b. from 0 to 60 percent of a component selected from the group consisting of natural or synthetic esters, natural or synthetic hydrocarbon fluids, or hydrocarbon oils derived from natural and petroleum sourced raw materials and combinations thereof; and
c. from 0.1 to 70 percent of additives selected from the group consisting of dispersants, anti-oxidants, anti-wear agents, anti-foam agents, corrosion inhibitors, detergents, seal-swell agents, viscosity improvers and combinations thereof.

14. The formulated lubricating oil of claim 11 comprising a mixture of
a. from 1 to 98 percent of said hydrogenated fluid having a 100° C kinematic viscosity of 2.5 to 4.6 centistokes; and
b. from 0 to 60 percent of a component selected from the group consisting of natural or synthetic esters, natural or synthetic hydrocarbon fluids, or hydrocarbon oils derived from natural and petroleum sourced raw materials and combinations thereof; and
c. from 0.1 to 70 percent of additives selected from the group consisting of dispersants, anti-oxidants, anti-wear agents, anti-foam agents, corrosion inhibitors, detergents, seal-swell agents, viscosity improvers and combinations thereof.

## Patentansprüche

1. Verfahren zur Herstellung einer Polyalphaolefinflüssigkeit, die vorbestimmte Eigenschaften aufweist, umfassend:
a. Umsetzen einer alpha-Olefin-Komponente, umfassend mindestens ein alpha-Olefin, das 30 bis 100 % 1-Tetradecen (C₁₄) enthält, in der Gegenwart eines Katalysatorsystems, bestehend aus BF₃ kombiniert mit einem Alkoholalkoxylat-Promotor, um ein umgesetztes Bodenprodukt und nicht umgesetzte restliche Monomere zu erzeugen;
b. Entfernen der nicht umgesetzten restlichen Monomere durch Destillation als Kopffraktion; und
c. Hydrieren von mindestens einem Teil des Bodenprodukts, um eine hydrierte Flüssigkeit mit einer kinematischen Viskosität bei 100 °C, bestimmt gemäß ASTM D-445, von 2,5 bis 4,6 Centistokes zu erhalten, und
wobei die Polyalphaolefinflüssigkeit einen Dimer-C₂₈-Gehalt von mehr als 70 % aufweist.

2. Verfahren nach Anspruch 1, wobei die alpha-Olefin-Komponente mehr als 90 % C₁₄-alpha-Olefin umfasst.

3. Verfahren nach Anspruch 1, wobei die hydrierte Polyalphaolefinflüssigkeit eine biologische Abbaubarkeit wie bestimmt durch den OECD-30-IB-Test von mindestens 50 Prozent aufweist.

4. Verfahren nach Anspruch 1, wobei die alpha-Olefin-Komponente ein erstes alpha-Olefin und ein zweites alpha-Olefin umfasst und wobei das erste alpha-Olefin C₁₄-alpha-Olefin ist und das zweite Olefin C₁₂-alpha-Olefin ist.

5. Verfahren nach Anspruch 4, wobei der C₁₄/C₁₂-Co-Dimer-Gehalt der Polyalphaolefinflüssigkeit größer als 80 % ist.

6. Verfahren nach Anspruch 1, wobei die alpha-Olefin-Komponente ein erstes alpha-Olefin und ein zweites alpha-Olefin umfasst und wobei das erste alpha-Olefin C₁₄-alpha-Olefin ist und das zweite Olefin ein Dimer von C₈-alpha-Olefin ist.

7. Verfahren nach Anspruch 6, wobei der C₁₄/C₁₆-Co-Dimer-Gehalt der Polyalphaolefinflüssigkeit größer als 80 % ist.

8. Verfahren nach Anspruch 1, wobei der Alkoholalkoxylat-Promotor in Schritt (a) 2-Methoxyethanol ist.

9. Verfahren nach Anspruch 1, wobei der Alkoholalkoxylat-Promotor in Schritt (a) 1-Methoxy-2-propanol ist.

10. Schmiermittelzusammensetzung, umfassend eine hydrierte Polyalphaolefinflüssigkeit, die durch das Verfahren nach einem der Ansprüche 1 bis 9 erhältlich ist und entweder
a. eine kinematische Viskosität bei 100 °C von 4 cSt, einen Gewichtsverlust durch Noack-Flüchtigkeit bei 250 °C von weniger als 19 % und einen Gewichtsverlust durch Noack-Flüchtigkeit bei 200 °C von weniger als 4 %, einen Viskositätsindex von mehr als 125, einen Stockpunkt niedriger als -35 °C und Cold-Crank-Simulator-Viskosität bei -35 °C von weniger als 1.500 cP aufweist, oder
b. eine kinematische Viskosität bei 100 °C von 3,4 cSt, einen Gewichtsverlust durch Noack-Flüchtigkeit bei 200 °C von 4 %, einen Stockpunkt von -50 °C und eine Brookfield-Viskosität bei -40 ° von weniger als 1.750 cP aufweist, oder
c. eine kinematische Viskosität bei 100 °C von 4 cSt, einen Gewichtsverlust durch Noack-Flüchtigkeit bei 250 °C von 13,9 %, einen Stockpunkt von -59 °C und eine Cold-Crank-Simulator-Viskosität bei -35 °C von weniger als 1.700 cP aufweist.

11. Formuliertes voll- oder teilsynthetisches Schmieröl, umfassend eine hydrierte Flüssigkeit, die eine kinematische Viskosität bei 100 °C von 2,5 bis 4,6 Centistokes aufweist, wobei die hydrierte Flüssigkeit eine Polyalphaolefinflüssigkeit ist, die durch das Verfahren nach einem der Ansprüche 1 bis 9 erhältlich ist.

12. Formuliertes Schmieröl nach Anspruch 11, umfassend eine Mischung aus:
a. 1 bis 97 Prozent der hydrierten Flüssigkeit, die eine kinematische Viskosität bei 100 °C von 2,5 bis 4,6 Centistokes aufweist; und
b. 0 bis 60 Prozent Flüssigkeiten ausgewählt aus der Gruppe bestehend aus synthetischen Estern, synthetischen Kohlenwasserstoffflüssigkeiten, Mineralölen, natürlichen Estern oder Kohlenwasserstoffölen, die von natürlichen und aus Erdöl stammenden Rohmaterialien stammen und Kombinationen davon; und
c. 0,1 bis 30 Prozent Additiven ausgewählt aus der Gruppe bestehend aus Dispergiermitteln, Antioxidantien, Antiverschleißmitteln, Antischaummitteln, Korrosionsinhibitoren, Detergenzien, Dichtungsquellmitteln, Viskositätsverbesserern und Kombinationen davon.

13. Formuliertes Schmieröl nach Anspruch 11, umfassend eine Mischung aus:
a. 1 bis 97 Prozent der hydrierten Flüssigkeit, die eine kinematische Viskosität bei 100 °C von 2,5 bis 4,6 Centistokes aufweist; und
b. 0 bis 60 Prozent einer Komponente ausgewählt aus der Gruppe bestehend aus natürlichen oder synthetischen Estern, natürlichen oder synthetischen Kohlenwasserstoffflüssigkeiten oder Kohlenwasserstoffölen, die von natürlichen und aus Erdöl stammenden Rohmaterialien stammen und Kombinationen davon; und
c. 0,1 bis 70 Prozent Additiven ausgewählt aus der Gruppe bestehend aus Dispergiermitteln, Antioxidantien, Antiverschleißmitteln, Antischaummitteln, Korrosionsinhibitoren, Detergenzien, Dichtungsquellmitteln, Viskositätsverbesserern und Kombinationen davon.

14. Formuliertes Schmieröl nach Anspruch 11, umfassend eine Mischung aus:
a. 1 bis 98 Prozent der hydrierten Flüssigkeit, die eine kinematische Viskosität bei 100 °C von 2,5 bis 4,6 Centistokes aufweist; und
b. 0 bis 60 Prozent einer Komponente ausgewählt aus der Gruppe bestehend aus natürlichen oder synthetischen Estern, natürlichen oder synthetischen Kohlenwasserstoffflüssigkeiten oder Kohlenwasserstoffölen, die von natürlichen und aus Erdöl stammenden Rohmaterialien stammen und Kombinationen davon; und
c. 0,1 bis 70 Prozent Additiven ausgewählt aus der Gruppe bestehend aus Dispergiermitteln, Antioxidantien, Antiverschleißmitteln, Antischaummitteln, Korrosionsinhibitoren, Detergenzien, Dichtungsquellmitteln, Viskositätsverbesserern und Kombinationen davon.

## Revendications

1. Procédé de production d'un fluide de polyalphaoléfine ayant des propriétés prédéterminées comprenant :
a. la réaction d'un composant alpha-oléfine comprenant au moins une alpha-oléfine contenant de 30 à 100 % de 1-tétradécène (C₁₄) en présence d'un système catalytique constitué de BF₃ combiné avec un promoteur d'alcoxylate d'alcool pour produire un produit de fond ayant réagi et des monomères résiduels n'ayant pas réagi ;
b. l'élimination des monomères résiduels n'ayant pas réagi par distillation sous forme de fraction de tête ; et
c. l'hydrogénation d'au moins une partie dudit produit de fond pour obtenir un fluide hydrogéné ayant une viscosité cinématique à 100 °C, déterminée selon la norme ASTM D-445 de 2,5 à 4,6 centistokes, et
dans lequel le fluide de polyalphaoléfine a une teneur en dimère en C₂₈ supérieure à 70 %.

2. Procédé selon la revendication 1, dans lequel le composant alpha-oléfine comprend plus de 90 % d'alpha-oléfine en C₁₄.

3. Procédé selon la revendication 1, dans lequel le fluide de polyalphaoléfine hydrogéné a une biodégradabilité, telle que déterminée par le test OECD 30 IB d'au moins 50 %.

4. Procédé selon la revendication 1, dans lequel le composant alpha-oléfine comprend une première alpha-oléfine et une seconde alpha-oléfine, et dans lequel la première alpha-oléfine est une alpha-oléfine en C₁₄ et la seconde oléfine est une alpha-oléfine en C₁₂.

5. Procédé selon la revendication 4, dans lequel la teneur en co-dimères en C₁₄/C₁₂ du fluide de polyalphaoléfine est supérieure à 80 %.

6. Procédé selon la revendication 1, dans lequel le composant alpha-oléfine comprend une première alpha-oléfine et une seconde alpha-oléfine, et dans lequel la première alpha-oléfine est une alpha-oléfine en C₁₄ et la seconde oléfine est un dimère d'alpha-oléfine en C₈.

7. Procédé selon la revendication 6, dans lequel la teneur en co-dimères en C₁₄/C₁₆ du fluide de polyalphaoléfine est supérieure à 80 %.

8. Procédé selon la revendication 1, dans lequel le promoteur d'alcoxylate d'alcool dans l'étape (a) est le 2-méthoxyéthanol.

9. Procédé selon la revendication 1, dans lequel le promoteur d'alcoxylate d'alcool dans l'étape (a) est le 1-méthoxy-2-propanol.

10. Composition lubrifiante comprenant un fluide de polyalphaoléfine hydrogéné pouvant être obtenu par le procédé selon l'une quelconque des revendications 1 à 9 et soit
a. ayant une viscosité cinématique à 100 °C de 4 cSt, une perte de poids de volatilité Noack à 250 °C inférieure à 19 % et une perte de poids de volatilité Noack à 200 °C inférieure à 4 un indice de viscosité supérieur à 125, un point d'écoulement inférieure à -35 °C et une viscosité de simulateur de démarrage à froid à -35 °C inférieure à 1 500 cP, ou
b. ayant une viscosité cinématique à 100 °C de 3,4 cSt, une perte de poids de volatilité Noack à 200 °C de 4 un point d'écoulement de -50 °C et une viscosité Brookfield à -40 ° inférieure à 1 750 cP ou
c. ayant une viscosité cinématique à 100 °C de 4 cSt, une perte de poids de volatilité Noack à 250 °C de 13,9 un point d'écoulement de -59 °C et une viscosité de simulateur de démarrage à froid à -35 °C inférieure à 1 700 cP.

11. Huile lubrifiante entièrement ou partiellement synthétique formulée comprenant un fluide hydrogéné ayant une viscosité cinématique à 100 °C de 2,5 à 4,6 centistokes, lequel fluide hydrogéné est un fluide de polyalphaoléfine pouvant être obtenu par le procédé selon l'une quelconque des revendications 1 à 9.

12. Huile lubrifiante formulée selon la revendication 11 comprenant un mélange :
a. de 1 à 97 % dudit fluide hydrogéné ayant une viscosité cinématique à 100 °C de 2,5 à 4,6 centistokes ; et
b. de 0 à 60 % de fluides choisis dans le groupe constitué d'esters synthétiques, de fluides d'hydrocarbures synthétiques, d'huiles minérales, d'esters naturels ou d'huiles d'hydrocarbures dérivés de matières premières naturelles et d'origine pétrolière et de leurs combinaisons ; et
c. de 0,1 à 30 % d'additifs choisis dans le groupe constitué de dispersants, d'antioxydants, d'agents anti-usure, d'agents anti-mousse, d'inhibiteurs de corrosion, de détergents, d'agents de gonflement des joints, d'agents améliorant la viscosité et de leurs combinaisons.

13. Huile lubrifiante formulée selon la revendication 11 comprenant un mélange :
a. de 1 à 97 % dudit fluide hydrogéné ayant une viscosité cinématique à 100 °C de 2,5 à 4,6 centistokes ; et
b. de 0 à 60 % d'un composant choisi dans le groupe constitué d'esters naturels ou synthétiques, de fluides d'hydrocarbures naturels ou synthétiques ou d'huiles d'hydrocarbures dérivés de matières premières naturelles et d'origine pétrolière et de leurs combinaisons ; et
c. de 0,1 à 70 % d'additifs choisis dans le groupe constitué de dispersants, d'antioxydants, d'agents anti-usure, d'agents anti-mousse, d'inhibiteurs de corrosion, de détergents, d'agents de gonflement des joints, d'agents améliorant la viscosité et de leurs combinaisons.

14. Huile lubrifiante formulée selon la revendication 11 comprenant un mélange :
a. de 1 à 98 % dudit fluide hydrogéné ayant une viscosité cinématique à 100 °C de 2,5 à 4,6 centistokes ; et
b. de 0 à 60 % d'un composant choisi dans le groupe constitué d'esters naturels ou synthétiques, de fluides d'hydrocarbures naturels ou synthétiques ou d'huiles d'hydrocarbures dérivés de matières premières naturelles et d'origine pétrolière et de leurs combinaisons ; et
c. de 0,1 à 70 % d'additifs choisis dans le groupe constitué de dispersants, d'antioxydants, d'agents anti-usure, d'agents anti-mousse, d'inhibiteurs de corrosion, de détergents, d'agents de gonflement des joints, d'agents améliorant la viscosité et de leurs combinaisons.
